# EUROPÄISCHE PATENTSCHRIFT

(11) **EP 2 502 604 B1**
(45) Veröffentlichungstag und Bekanntmachung des Hinweises auf die Patenterteilung: **25.09.2013**
(21) Anmeldenummer: 11159050.1
(22) Anmeldetag: 21.03.2011
(51) Int. Cl.: A61F 2/34, A61F 2/30

(54) **Gelenkpfannenimplantat**
Joint socket implant
Implant de poêle articulée

(43) Veröffentlichungstag der Anmeldung: 26.09.2012
(73) Patentinhaber: Jossi Holding AG, 8546 Islikon (CH)
(72) Erfinder: Gugler, Christian, 8500 Frauenfeld (CH); Schmidt, Martin, 9545 Wängi (CH); Meyenhofer, Andreas, 8255 Schlattingen (CH)
(74) Vertreter: Hepp Wenger Ryffel AG

(56) Entgegenhaltungen:
- EP-A1- 2 338 443
- EP-B1- 0 839 016
- WO-A1-97/39702
- DE-A1- 2 259 313
- DE-A1- 10 106 863
- DE-C1- 3 535 959
- GB-A- 2 268 408

## Beschreibung

Die vorliegende Erfindung betrifft ein Gelenkpfannenimplantat gemäss dem Oberbegriff von Anspruch 1.

Zur Besseren Verankerung von Implantaten, insbesondere Endoprothesen in Knochen sind eine Vielzahl von Oberflächenstrukturen bekannt. Diese reichen von aufgerauten Oberflächen bis zur Ausstattung der Oberfläche mit komplexen Strukturelementen.

Insbesondere im Bereich der Hüftgelenksprothesen ist es von Vorteil, wenn die Implantatteile, vor allem die im Hüftknochen angebrachte Gelenkpfanne, ausreiss- und drehfest im Knochen verankert werden können. Während eine Gruppe von Gelenkpfannen durch Schrauben verankert wird, erfolgt bei einer zweite Gruppe die Befestigung mittels Knochenzement.

Um das Ausreissverhalten sowie die Drehfestigkeit einer mittels Knochenzement befestigten Gelenkpfanne zu erhöhen, wird die Oberfläche solcher Pfannen üblicherweise mit Strukturelementen versehen. Diese Strukturelemente verteilen dabei auftretende Kräfte gleichmässig und verhindern so eine Drehung oder gar das Ausreissen der Gelenkpfanne.

Dem Fachmann ist eine Vielzahl an verschiedenen Oberflächenstrukturen bekannt. Die Oberflächenstrukturen werden dabei meistens durch spanabhebende Verfahren in die Oberfläche der Implantate eingearbeitet.

Die EP 0 839 016 offenbart beispielsweise eine Hüftgelenkpfanne mit einer Oberflächenstruktur mit rhombenförmigen Erhebungen. Die Erhebungen werden durch sich überkreuzende, schraubenlinienförmige Gewinde mit entgegengesetzter Steigung begrenzt. Die Tiefe der Gewinde nimmt dabei vom Äquator zum Pol hin ab.

Die DE 101 06 863 beschreibt eine implantierbare Hüftpfanne welche an der Oberfläche eine Struktur in Form von Pyramidenspitzen aufweist. Die Pyramidenspitzen werden durch spanabhebendes Einarbeiten von Rechtsgewinde-Profilen und Linkgsgewinde-Profilen erzeugt. Die Profilhöhe dieser Gewindeprofile nimmt von der Eingangsebene der Pfanne gegen deren Polbereich hin ab.

Durch die Kombination von Gewinde und Kugelkalotte nimmt die Anzahl der Erhebungen gegen den Pol hin stark ab, was die Verankerung der Gelenkspfanne im Knochen reduziert. Ferner wird durch die abnehmende Tiefe der Gewinde die Verankerung im Knochen zusätzlich geschwächt.

Die GB 2 268 408 zeigt ein Gelenkpfannenimplantat gemäß dem Oberbegriff des Anspruchs 1.

Eine Aufgabe der vorliegenden Erfindung ist es, eine Oberflächenstruktur für Gelenkpfannenimplantat zu schaffen, welche die Nachteile des Bekannten überwindet und welche insbesondere eine gute Verankerung der Gelenkpfanne im Knochen ermöglicht und einfach herzustellen ist. Diese Aufgabe wird mit einem Gelenkpfannenimplantat gemäss Anspruch 1 gelöst.

Das erfindungsgemässe Gelenkpfannenimplantat hat die Form einer Kugelkalottenschale und weist mindestens einen Bereich mit einer Oberflächenstruktur auf. Der mindestens eine Bereich erstreckt sich zwischen dem Äquator und dem Pol der Kugelkalottenschale. Die Oberflächenstruktur umfasst eine Vielzahl von Strukturelementen, welche durch sich überkreuzende Rillen mit entgegengesetzter Steigung gebildet sind. Diese Rillen weisen eine Krümmung auf, welche derart ausgebildet ist, dass die Anzahl der Strukturelemente auf dem Äquator oder einem äquatornahen Breitenkreis gleich ist wie auf einem polnahen Breitenkreis.

Das erfindungsgemässe Gelenkpfannenimplantat ist vorzugsweise ein Hüftgelenkpfannenimplantat. Das Gelenkpfannenimplantat ist bevorzugterweise in der Form einer Kugelkalottenschale, besonders bevorzugt in der Form eine Halbkugelschale.

Die Kugelkalottenschale weist eine äussere, konvexe Oberfläche auf, welche bei der im Knochen eingesetzten Gelenkpfanne auf dem Knochen anliegt. Weiter weist die Kugelkalottenschale eine innere, konkave oder annähernd konkave Oberfläche auf, in die eine Lagerschale eingesetzt werden kann. Zwischen diesen beiden Oberflächen weist das Gelenkpfannenimplantat eine Wand auf, deren Dicke vorzugsweise überall gleich ist, d.h. die Radien der äusseren wie auch der inneren Oberfläche haben den gleichen Mittelpunkt und sind beide konstant. Alternativ kann sich die Dicke der Wand auch verändern, z.B. kann die innere Oberfläche aus mehreren in spezifischen Winkel zueinander stehenden Flächen bestehen.

Die Grösse der Kugelkalottenschale kann variieren, je nach Patient für den das Gelenkpfannenimplantat vorgesehen ist oder je nach Anwendungsort, beispielsweise am Hüft- oder Schultergelenk.

Ferner weist das Gelenkpfannenimplantat mindestens einen Bereich mit einer Oberflächenstruktur auf. Dieser Bereich befindet sich zwischen dem Äquator und dem Pol und erstreckt sich bevorzugterweise um den gesamten Umfang des Gelenkpfannenimplantats. Je nach Ausgestaltung weist der Bereich unterschiedliche Ausdehnungen zwischen Äquator und Pol des Gelenkpfannenimplantats auf. Besonders bevorzug erstreckt sich dieser Bereich vom Äquator in Richtung Pol über mindestens die Hälfte der Oberfläche.

Als "Äquator" im Sinne der Anmeldung wird die Öffnung der Kugelkalottenschale verstanden, während als "Pol" diejenige Stelle bezeichnet wird, welche dem Apex der Kugelkalottenschale entspricht.

Ein "äquatornaher Bereich" im Sinne der vorliegenden Anmeldung liegt näher am Äquator als am Pol, während ein "polnaher Bereich" näher beim Pol als beim Äquator des Gelenkpfannenimplantats liegt.

Die Oberflächenstruktur umfasst eine Vielzahl von Strukturelementen. Die Strukturelemente werden durch sich überkreuzende Rillen gebildet. Die Rillen sind bevorzugt durch ein spanabhebendes Verfahren in der Oberfläche erzeugt worden, wie beispielsweise durch Fräsen.

Die Rillen weisen bevorzugt entgegengesetzte Steigungen auf. D.h. dass in Bezug auf den Äquator einzelne Rillen nach Links verlaufen während andere nach Rechts verlaufen. Jedes Strukturelement ist dabei von zwei nach Links und zwei nach Rechts verlaufenden Rillen begrenzt. Die Grundfläche der Strukturelemente weist damit eine rhombenähnliche Form auf.

Die Rillen weisen besonders bevorzugt eine Krümmung auf, welche derart ausgebildet ist, dass die Anzahl der Strukturelemente auf dem Äquator oder einem äquatornahen Breitenkreis gleich ist wie auf einem polnahen Breitenkreis. Die Anzahl der Strukturelemente auf jedem beliebigen Breitenkreis des Gelenkpfannenimplantats ist daher gleich.

Die Rillen mit der gleichen Steigungsrichtung weisen alle dieselbe Krümmung auf. Damit die Anzahl der Strukturelemente auf dem Äquator oder einem äquatornahen Breitenkreis gleich ist wie auf einem polnahen Breitenkreis, nimmt die Steigung der Rillen gegenüber den Breitenkreisen und somit der Winkel zwischen einer Tangente der Rillenkrümmung und einem Breitenkreis vom Äquator zum Pol hin kontinuierlich zu. Gleichzeitig wird der Radius der Krümmung vom Äquator zum Pol hin kontinuierlich kleiner. Dabei nimmt der Abstand zwischen zwei Rillen mit gleicher Steigungsrichtung vom Äquator in Richtung Pol hin ab.

Die Grundfläche der Strukturelemente vergrössert sich dadurch vom Pol zum Äquator hin in der Richtung parallel zu den Breitengraden. Die Strukturelemente, welche auf dem gleichen Meridian liegen, weisen daher eine vom Pol in Richtung Äquator stetig grösser werdende Ausdehnung parallel zu den Breitenkreisen auf. Da die Gelenkpfanne in einer Richtung rechtwinklig zu den Breitenkreisen in den Knochen eingesetzt wird, hat dies den Vorteil, dass sich jedes Strukturelement in Richtung parallel zu den Breitenkreisen ein bisschen weiter in den Knochen schneiden muss, was die Verankerung des Gelenkpfannenimplantats im Knochen erhöht.

Bevorzugt weisen die Rillen vom äquatornahen Bereich zum polnahen Bereich hin eine konstante Tiefe auf. Dadurch weisen auch die Strukturelemente eine konstante Höhe auf. Dadurch ist die Verankerung über der gesamten Oberfläche des Gelenkpfannenimplantats gleich gut. Ausserdem ermöglicht eine solche Konfiguration eine gleich gute Stabilität des Gelenkpfannenimplantats im Knochen entlang der Polachse wie auch gegen Rotation und Verkippen.

Die Strukturelemente sind besonders bevorzugt in der Form einer Pyramide oder eines Pyramidenstumpfes. Die Form der Strukturelemente ist durch das Profil der Rillen vorgegeben, so dass sich durch geeignete Wahl des Rillenprofils die Form der Strukturelemente verändern lässt. Alternativ weisen die Strukturelemente eine beliebige polyedrische oder auch gerundete Form auf. Nach dem Erzeugen der Strukturelemente durch die Rillen können diese noch nachbearbeitet werden, z.B. durch Fräsen.

Bevorzugt sind die Strukturelemente im meridianen Querschnitt in Richtung Äquator geneigt. D.h. dass mindestens diejenige Seite des Strukturelements, welche in Richtung Äquator zeigt überhängt, also in einem Winkel von grösser als 90° zum Grund der Rille steht. Dies erhöht die zum Ausreissen des Gelenkpfannenimplantats nötige Kraft zusätzlich.

Bevorzugt weist das Gelenkpfannenimplantat mindestens einen zweiten Bereich mit einer Oberflächenstruktur auf. Die beiden Oberflächenstrukturen weisen dabei eine unterschiedliche Anzahl von Strukturelementen auf und/oder die Strukturelemente unterscheiden sich in ihrer Konfiguration. Ferner bevorzugt kann das Gelenkpfannenimplantat auch mehr als zwei Bereiche mit unterschiedlichen Oberflächenstrukturen aufweisen, wie zum Beispiel drei, vier, fünf oder mehr Bereiche. Die Bereiche können alle unterschiedliche Ausdehnungen zwischen Äquator und Pol des Gelenkpfannenimplantats aufweisen oder aber alle die gleiche Ausdehnung. Dadurch kann das Gelenkpfannenimplantat mit Bereichen ausgestattet werden, welche an spezifische Knochenbereiche angepasste Verankerungseigenschaften aufweisen.

Der mindestens eine Bereich mit einer Oberflächenstruktur befindet sich bevorzugterweise auf der konvexen Aussenfläche und/oder der konkaven Innenfläche des Gelenkpfannenimplantats. Eine Oberflächenstruktur auf der konvexen Aussenfläche des Gelenkpfannenimplantats ermöglicht die Verankerung des Gelenkpfannenimplantats im Knochen. Eine Oberflächenstruktur auf der konkaven Innenfläche ermöglicht eine gute Verbindung zwischen dem Gelenkpfannenimplantat und einer Lagerschale.

Besonders bevorzugt umfasst ein erfindungsgemässes Gelenkpfannenimplantat einen ersten Bereich mit einer ersten Oberflächenstruktur mit Strukturelementen mit im meridianen Querschnitt in Richtung Äquator geneigten Oberflächenstrukturen in einem äquatornahen Bereich und einen weiteren Bereich mit einer zweiten Oberflächenstruktur mit Strukturelementen in Form von Pyramiden in einem polnahen Bereich.

Eine derartige Konfiguration des Gelenkpfannenimplantats hat den Vorteil, dass der äquatornahe Bereich durch die sägezahnförmigen Strukturelemente die zum Ausreissen des eingesetzten Gelenkpfannenimplantats zusätzlich erhöht, während die pyramidenförmigen Strukturelemente im polnahen Bereich das Gelenkpfannenimplantat optimal vor dem Verdrehen und/oder Verkippen sichern.

Bevorzugterweise bilden die Rillen in einem äquatornahen Bereich der Oberflächenstruktur mit einem Breitenkreis einen Winkel von 0° bis 90°. Bevorzugt liegt dieser Winkel in einem Bereich von 15° bis 75°. Durch die Krümmung der Rillen vergrössert sich dieser Winkel zum Pol hin kontinuierlich.

Der mindestens eine Bereich mit einer Oberflächenstruktur weist bevorzugt auf dem Äquator oder dem äquatornahen Breitenkreis sowie dem polnahen Breitenkreis 10 bis 100 Strukturelemente auf.

Erfindungsgemäß weisen die Strukturelemente eine Grundfläche auf, welche annähernd rhombenförmig ist. Im Unterschied zu einem Rhombus sind die Seitenkanten, d.h. die die Grundfläche begrenzenden Rillen nicht gerade sondern weisen eine Krümmung auf. Durch die Krümmung der Rillen nimmt die Länge der zu den Meridianen parallelen Diagonalen der Grundflächen der Strukturelemente vom Äquator zum Pol hin zu während die zu den Breitenkreisen parallelen Diagonalen abnehmen. D.h. dass ein Strukturelement je nach Nähe zum Äquator beziehungsweise zum Pol eine unterschiedliche Grundfläche aufweist.

Die Strukturelemente, welche näher beim Pol sind, weisen eine Grundfläche auf, welche in Richtung der Meridiane länger ist als in Richtung der Breitenkreise. Beim Einsetzen der Gelenkpfanne in einen Knochen kommen diese Strukturelemente als erste mit dem Knochen in Kontakt. Die Form von deren Grundflächen erleichtert dabei das Einsetzen des Gelenkpfannenimplantats, da sich die Strukturelemente leicht in den Knochen einschneiden können.

Die unterschiedlichen Geometrien der Grundflächen der Strukturelemente weist zudem den Vorteil auf, dass diejenigen Strukturelemente, welche eine grössere Ausdehnung in Richtung der Meridiane aufweisen das Gelenkpfannenimplantat gut gegen Verdrehen und Verkippen sichern, während diejenigen Strukturelemente, welche in Richtung der Breitenkreise eine grössere Ausdehnung aufweisen, das Gelenkpfannenimplantat besser gegen das Ausreissen sichern.

Bevorzugterweise weisen die Rillen einen symmetrischen oder asymmetrischen Querschnitt auf. Dadurch lässt sich die Form der Strukturelemente zusätzlich verändern.

Weitere Aspekte sowie Details der Erfindung ergeben sich aus der folgenden Beschreibung von Beispielen und Figuren. Es zeigen:
- Fig. 1:: eine Aufsicht eines Zwischenerzeugnisses bei der Produktion eines Gelenkpfannenimplantats mit Rillen;

- Fig. 2:: eine Seitenansicht des Zwischenerzeugnisses der Fig. 1;
- Fig. 3:: eine Aufsicht eines erfindungsgemässen Gelenkpfannenimplantats mit einem Bereich mit einer Oberflächenstruktur;
- Fig. 4:: eine Seitenansicht des Gelenkpfannenimplantats der Figur 3;
- Fig. 5:: eine Aufsicht eines Gelenkpfannenimplantats mit zwei Bereichen mit einer Oberflächenstruktur;
- Fig. 6:: eine Seitenansicht des Gelenkpfannenimplantats der Fig. 4;
- Fig. 7:: einen Teilschnitt durch das Gelenkpfannenimplantat der Figur 4; und
- Fig. 8:: eine Projektion der Oberfläche des Gelenkpfannenimplantats der Figur 3.

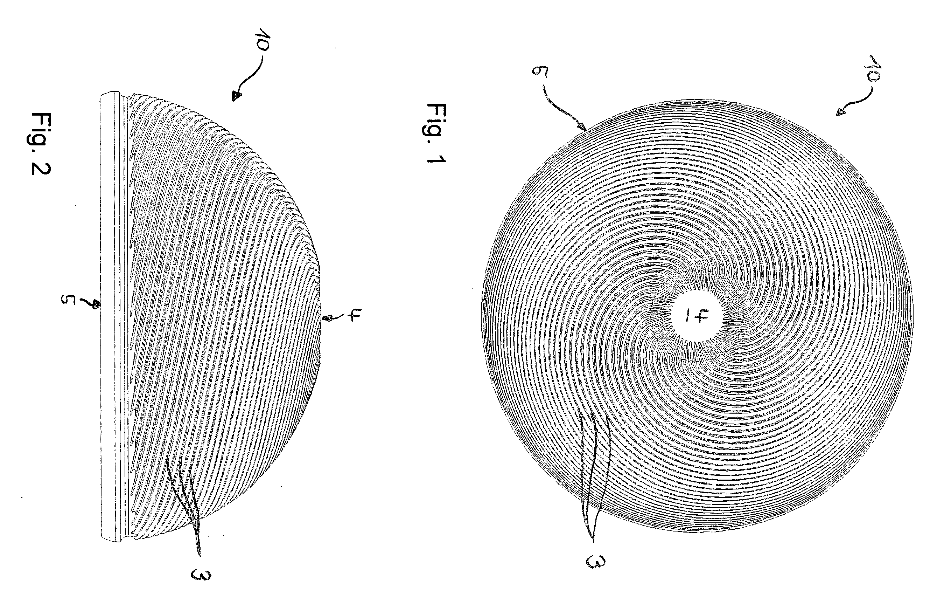

Die Figur 1 zeigt eine Aufsicht auf ein Zwischenerzeugnis 10 bei der Herstellung eines Gelenkpfannenimplantat 1 mit nur nach Links gerichteten Rillen 3. Die Figur 2 zeigt eine Seitenansicht desselben Zwischenerzeugnisses 10. Das Zwischenerzeugnis 10 ist in der Form einer Kugelkalottenschale und weist einen Pol 4 sowie einen Äquator 5 auf. Bei diesem Ausführungsbeispiel entspricht die Kugelkalottenschale einer Halbkugeschale. Das Zwischenerzeugnis 10 ist das Ergebnis eines ersten Bearbeitungsschrittes bei der Herstellung einer erfindungsgemässen Gelenkpfannenimplantats, bei dem zunächst Rillen 3 in nur einer Steigungsrichtung eingearbeitet werden.

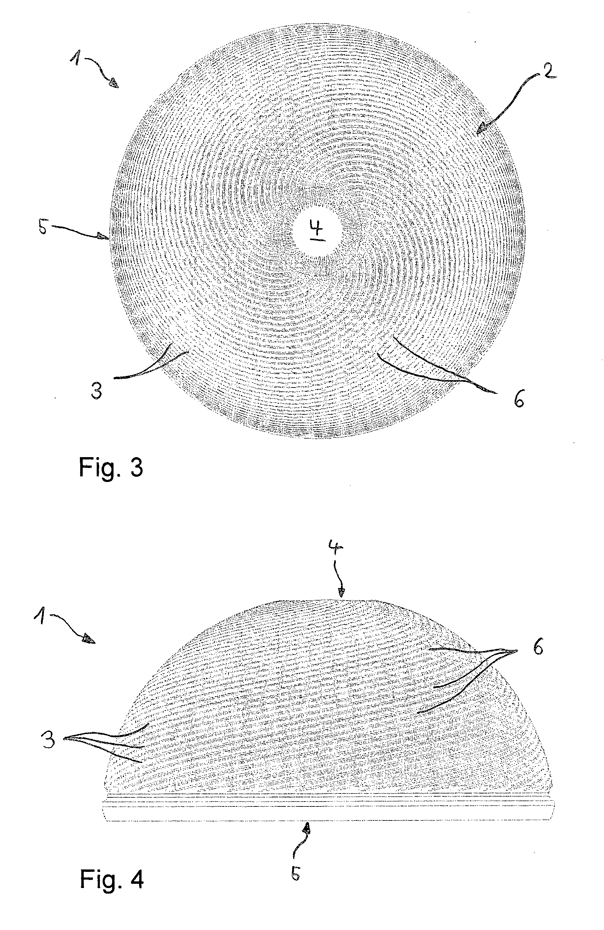

Die Figuren 3 und 4 zeigen eine Auf- sowie eine Seitenaufsicht eines Gelenkpfannenimplantats 1 mit einem Bereich mit einer Oberflächenstruktur 2. Die Oberflächenstruktur 2 weist eine Vielzahl von Strukturelementen 6 auf, welche durch die sich überkreuzenden Rillen 3 gebildet sind. Die Rillen 3 weisen eine Krümmung auf, die derart gewählt ist, dass die Anzahl der Strukturelemente 6 auf dem Äquator 5 oder einem äquatornahen Breitenkreis gleich ist wie in einem polnahen Breitenkreis. Die gebildeten Strukturelemente weisen eine annähernd rhombenförmige Grundlfäche auf, welche durch die gekrümmten Rillen 3 begrenzt wird.

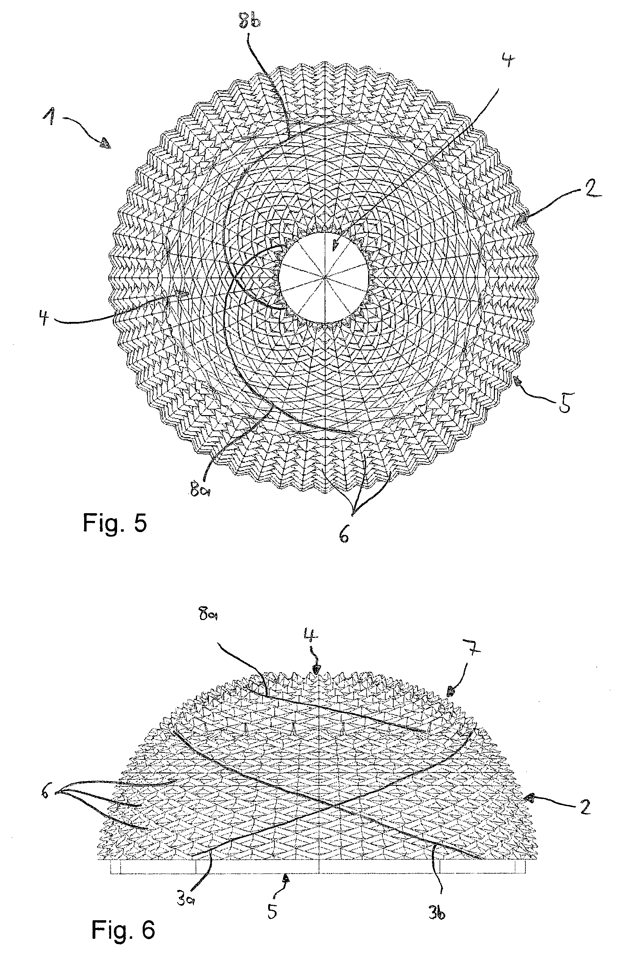

Die Figuren 5 und 6 zeigen eine Auf- und Seitenansicht einer besonders bevorzugten Ausführungsform des erfindungsgemässen Gelenkpfannenimplantats 1. Dieses Gelenkpfannenimplantat 1 umfasst zwei Bereiche mit einer Oberflächenstruktur 2, 7. Die Rillen 3a, 3b der ersten Oberflächenstruktur 2 unterscheiden sich von den Rillen 8a, 8b der zweiten Oberflächenstruktur. Beispielsweise kann die Anzahl der Rillen, deren Krümmungsverlauf, Profil und/oder Tiefe unterschiedlich sein. Dadurch ergeben sich in den beiden Bereichen 2, 7 auch unterschiedliche Formen der Strukturelemente 6. Auf diesen Figuren ist der Krümmungsverlauf der Rillen 3a, 3b, 8a, 8b gut ersichtlich. Ebenso ersichtlich ist die Veränderung der Grundflächen der Strukturelemente 6. Dabei nimmt die Länge der Diagonalen parallel zu den Breitenkreisen vom Äquator in Richtung Pol hin ab, während die Länge der Diagonalen parallel zu den Meridianen zunimmt.

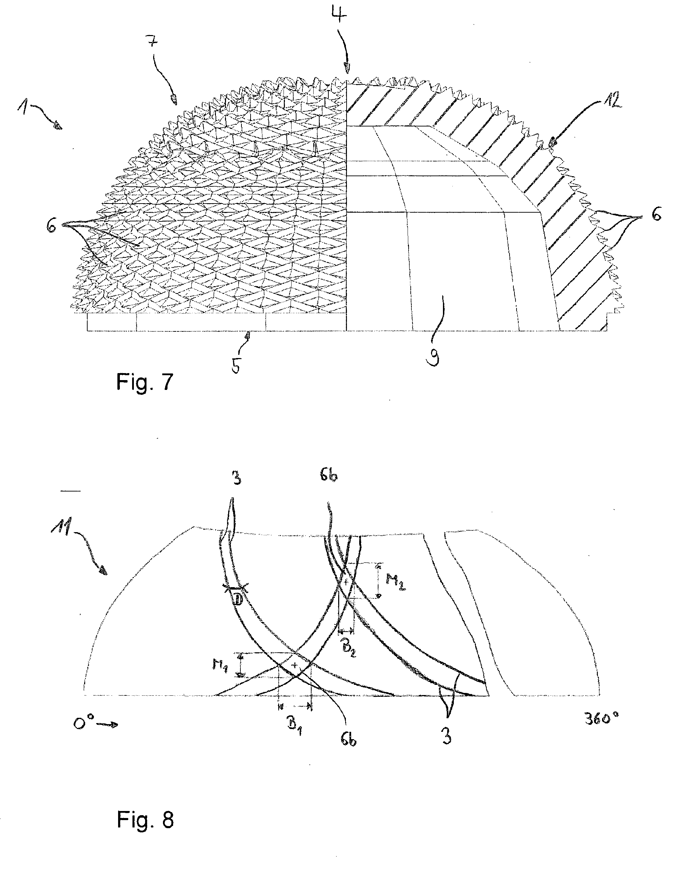

Die Figur 7 zeigt einen Teilschnitt durch ein Gelenkpfannenimplantat 1. Wie auf dieser Figur ersichtlich, weist das Gelenkpfannenimplantat 1 eine annähernd konkave Innenfläche 9 auf, welche aus mehreren, in einem Winkel zueinander stehenden Flächen besteht. Ferner weist das Gelenkpfannenimplantat eine konvexe Aussenfläche 12 auf. Bei dieser Ausführungsform weist nur die konvexe Aussenfläche 12 eine Oberflächenstruktur mit Strukturelementen 6 auf.
Auf der Figur 8 ist eine Projektion der Oberfläche 11 eines erfindungsgemässen Gelenkpfannenimplantats dargestellt. Gut ersichtlich ist die Krümmung der Rillen 3, welche derart ausgestaltet ist, dass auf dem Äquator oder einem äquatornahen Breitenkreis die gleiche Anzahl an Strukturelementen 6 liegen wie auf einem polnahen Breitenkreis. Die Krümmung der Rillen 3 führt dazu, dass sich die Form der Grundflächen der Strukturelement 6 vom Äquator in Richtung Pol verändern. Dies ist auf der Figur beispielhaft an zwei Strukturelementen 6a, 6b mit annähernd rhombenförmigen Grundflächen gezeigt. Die Grundfläche des äquatornahen Strukturelements 6a weist eine Diagonale B₁ parallel zu den Breitenkreisen auf, welche länger ist als die zu den Meridianen parallele Diagonale M₁. Mit zunehmendem Abstand zum Äquator verändert sich dieses Längenverhältnis der Diagonalen. Das polnahe Strukturelement 6b weist eine zu den Breitenkreisen parallele Diagonale B₂ auf, die kürzer ist als die zu den Meridianen parallele Diagonale M₂. Die Länge der zu den Breitenkreisen parallele Diagonale B₂ des polnahen Strukturelements 6b ist zudem kürzer als die entsprechende Diagonale B₁ des äquatornahen Strukturelements 6a, während die zu den Meridianen parallele Diagonale M₂ des polnahen Strukturelements 6b länger ist als die entsprechende Diagonale M₁ des äquatornahen Strukturelements 6a. Ferner ist auch ersichtlich, dass der Abstand D zwischen zwei Rillen mit gleicher Steigung vom Äquator zum Pol hin abnimmt.

## Patentansprüche

1. Gelenkpfannenimplantat (1), insbesondere Hüftgelenkpfannenimplantat in Form einer Kugelkalottenschale mit mindestens einem Bereich zwischen dem Aquator und dem Pol der Kugelkalottenschale mit einer Oberflächenstruktur (2,7), welche eine Vielzahl von Strukturelementen (6) umfasst, welche durch sich überkreuzende Rillen (3,8) mit entgegengesetzter Steigung gebildet sind, wobei die Rillen (3, 8) eine Krümmung aufweisen, welche derart ausgebildet ist, dass die Anzahl der Strukturelemente (6) auf dem Äquator (5) oder auf einem äquatornahen Breitenkreis gleich ist wie auf einem polnahen Breitenkreis, wobei die Strukturelemente (6) einen annähernd rhombenförmigen Grundriss aufweisen und die Länge einer Diagonalen (B₁, B₂) des Grundrisses parallel zu den Breitenkreisen vom Äquator (5) Richtung Pol (4) abnimmt, **dadurch gekennzeichnet, dass** die Länge einer Diagonalen (M₁, M₂) des Grundrisses parallel zu den Meridianen vom Äquator (5) Richtung Pol (4) zunimmt.

2. Gelenkpfannenimplantat (1) nach Anspruch 1, **dadurch gekennzeichnet, dass** die Rillen (3,8) vom Äquator (5) zum Pol (4) hin eine konstante Tiefe aufweisen.

3. Gelenkpfannenimplantat (1) nach einem der Ansprüche 1 oder 2, **dadurch gekennzeichnet, dass** die Strukturelemente (6) pyramidenförmig oder pyramidenstumpfförmig ausgebildet sind.

4. Gelenkpfannenimplantat (1) nach einem der Ansprüche 1 bis 3, **dadurch gekennzeichnet, dass** die Strukturelemente (6) im meridianen Querschnitt in Richtung des Äquators (5) geneigt sind.

5. Gelenkpfannenimplantat (1) nach einem der Ansprüche 1 bis 4, **dadurch gekennzeichnet, dass** zwischen dem Äquator (5) und dem Pol (4) wenigstens zwei Bereiche mit einer Oberflächenstruktur (2,7) mit unterschiedlicher Anzahl und/oder unterschiedlicher Konfiguration der Strukturelemente (6) angeordnet sind.

6. Gelenkpfannenimplantat (1) nach einem der Ansprüche 1 bis 5, **dadurch gekennzeichnet, dass** sich der mindestens eine Bereich mit einer Oberflächenstruktur (2,7) auf der konvexen Aussenfläche (12) und/oder der konkaven Innenfläche (9) des Gelenkpfannenimplantats (1) befindet.

7. Gelenkpfannenimplantat (1) nach einem der Ansprüche 5 oder 6, **dadurch gekennzeichnet, dass** das Gelenkpfannenimplantat (1) einen ersten Bereich mit einer ersten Oberflächenstruktur (2) mit Strukturelementen (6), welche im meridianen Querschnitt gegen den Äquator (5) geneigt sind in einem äquatornahen Bereich und einen weiteren Bereich mit einer zweiten Oberflächenstruktur (7) mit Strukturelementen (6) in Form von Pyramiden oder Pyramidenstümpfen in einem polnahen Bereich aufweist.

8. Gelenkpfannenimplantat (1) nach einem der Ansprüche 1 bis 7, **dadurch gekennzeichnet, dass** die Rillen (3,8) in einem äquatornahen Bereich der Oberflächenstruktur (2,7) mit einem Breitenkreis einen Winkel von 0° bis 90°, bevorzugt von 15° bis 75° bilden, wobei sich dieser Winkel zum Pol hin kontinuierlich vergrössert.

9. Gelenkpfannenimplantat (1) nach einem der Ansprüche 1 bis 8, **dadurch gekennzeichnet, dass** der mindestens eine Bereich mit einer Oberflächenstruktur (2,7) auf dem Äquator (5) oder dem äquatornahen Bereich sowie dem polnahen Bereich 10 bis 100 Strukturelemente (6) aufweist.

10. Gelenkpfannenimplantat (1) nach einem der Ansprüche 1 bis 9, **dadurch gekennzeichnet, dass** die Rillen (3,8) einen symmetrischen oder asymmetrischen Querschnitt aufweisen.

## Claims

1. Joint socket implant (1), in particular a hip-joint socket implant, in the form of a spherical cap shell with at least one region between the equator and the pole of the spherical cap shell having a surface structure (2, 7) comprising a multiplicity of structural elements (6), which are formed by intersecting grooves (3, 8) with opposite pitch, wherein the grooves (3, 8) have a curvature designed in such a way that the number of the structural elements (6) on the equator (5) or on a circle of latitude near the equator is the same as on a circle of latitude near the pole, wherein the structural elements (6) have an approximately rhomboidal outline, and the length of a diagonal (B₁, B₂) of the outline parallel to the circles of latitude decreases from the equator (5) in the direction of the pole (4), **characterized in that** the length of a diagonal (M₁, M₂) of the outline parallel to the meridians increases from the equator (5) in the direction of the pole (4).

2. Joint socket implant (1) according to Claim 1, **characterized in that** the grooves (3, 8) have a constant depth from the equator (5) to the pole (4).

3. Joint socket implant (1) according to either of Claims 1 and 2, **characterized in that** the structural elements (6) have the shape of a pyramid or of a truncated pyramid.

4. Joint socket implant (1) according to one of Claims 1 to 3, **characterized in that** the structural elements (6), in the meridian cross section, are inclined in the direction of the equator (5).

5. Joint socket implant (1) according to one of Claims 1 to 4, **characterized in that** at least two regions having a surface structure (2, 7) with a different number and/or different configuration of the structural elements (6) are arranged between the equator (5) and the pole (4).

6. Joint socket implant (1) according to one of Claims 1 to 5, **characterized in that** the at least one region having a surface structure (2, 7) is located on the convex outer face (12) and/or the concave inner face (9) of the joint socket implant (1).

7. Joint socket implant (1) according to either of Claims 5 and 6, **characterized in that** the joint socket implant (1) has, in a region near the equator, a first region having a first surface structure (2) with structural elements (6) which, in the meridian cross section, are inclined towards the equator (5), and, in a region near the pole, it has a further region having a second surface structure (7) with structural elements (6) in the shape of pyramids or truncated pyramids.

8. Joint socket implant (1) according to one of Claims 1 to 7, **characterized in that** the grooves (3, 8), in a region of the surface structure (2, 7) near the equator, form an angle of 0° to 90°, preferably of 15° to 75°, to a circle of latitude, this angle increasing continuously towards the pole.

9. Joint socket implant (1) according to one of Claims 1 to 8, **characterized in that** the at least one region having a surface structure (2, 7) on the equator (5), or the region near the equator, and on the region near the pole, has 10 to 100 structural elements (6).

10. Joint socket implant (1) according to one of Claims 1 to 9, **characterized in that** the grooves (3, 8) have a symmetrical or asymmetrical cross section.

## Revendications

1. Implant cotyloïdien (1), en particulier implant pour l'articulation de la hanche, sous la forme d'une coque à calotte sphérique comprenant au moins une région entre l'équateur et le pôle de la coque à calotte sphérique, comprenant une structure de surface (2, 7) qui comprend une pluralité d'éléments structurels (6) qui sont formés par des rainures entrecroisées (3, 8) de pas opposé, les rainures (3, 8) présentant une courbure qui est réalisée de telle sorte que le nombre des éléments structurels (6) sur l'équateur (5) ou sur un parallèle proche de l'équateur soit égal à ceux sur un parallèle proche du pôle, les éléments structurels (6) présentant un pourtour approximativement rhomboïde et la longueur d'une diagonale (B₁, B₂) du pourtour diminuant parallèlement aux parallèles depuis l'équateur (5) dans la direction du pôle (4), **caractérisé en ce que** la longueur d'une diagonale (M₁, M₂) du pourtour augmente parallèlement aux méridiens depuis l'équateur (5) en direction du pôle (4).

2. Implant cotyloïdien (1) selon la revendication 1, **caractérisé en ce que** les rainures (3, 8) présentent une profondeur constante depuis l'équateur (5) jusqu'au pôle (4).

3. Implant cotyloïdien (1) selon l'une quelconque des revendications 1 ou 2, **caractérisé en ce que** les éléments structurels (6) sont réalisés en forme de pyramides ou en forme de troncs de pyramide.

4. Implant cotyloïdien (1) selon l'une quelconque des revendications 1 à 3, **caractérisé en ce que** les éléments structurels (6) sont inclinés dans la direction de l'équateur (5) en section transversale méridienne.

5. Implant cotyloïdien (1) selon l'une quelconque des revendications 1 à 4, **caractérisé en ce qu'**entre l'équateur (5) et le pôle (4) sont disposés au moins deux régions ayant une structure de surface (2, 7) ayant un nombre différent et/ou une configuration différente des éléments structurels (6).

6. Implant cotyloïdien (1) selon l'une quelconque des revendications 1 à 5, **caractérisé en ce que** l'au moins une région avec une structure de surface (2, 7) se trouve sur la surface extérieure convexe (12) et/ou sur la surface intérieure concave (9) de l'implant cotyloïdien (1).

7. Implant cotyloïdien (1) selon l'une quelconque des revendications 5 ou 6, **caractérisé en ce que** l'implant cotyloïdien (1) présente une première région avec une première structure de surface (2) avec des éléments structurels (6) qui sont inclinés vers l'équateur (5) en section transversale méridienne et dans une région proche de l'équateur et une région supplémentaire avec une deuxième structure de surface (7) avec des éléments structurels (6) en forme de pyramides ou de troncs de pyramide dans une région proche du pôle.

8. Implant cotyloïdien (1) selon l'une quelconque des revendications 1 à 7, **caractérisé en ce que** les rainures (3, 8) forment, dans une région proche de l'équateur de la structure de surface (2, 7) avec un parallèle, un angle de 0° à 90°, de préférence de 15° à 75°, cet angle augmentant en continu vers le pôle.

9. Implant cotyloïdien (1) selon l'une quelconque des revendications 1 à 8, **caractérisé en ce que** l'au moins une région avec une structure de surface (2, 7) sur l'équateur (5) ou sur la région proche de l'équateur ainsi que la région proche du pôle présente 10 à 100 éléments structurels (6).

10. Implant cotyloïdien (1) selon l'une quelconque des revendications 1 à 9, **caractérisé en ce que** les rainures (3, 8) présentent une section transversale symétrique ou asymétrique.
